# EUROPEAN PATENT APPLICATION

(11) **EP 1 111 040 A1**
(43) Date of publication of application: **27.06.2001**
(21) Application number: 99204245.7
(22) Date of filing: 10.12.1999
(51) Int. Cl.: C12N 5/10

(54) **Infection of eukaryotic cells with viruses in vitro**

(71) Applicant: Favre, Daniel, 1205 Genève (CH)
(72) Inventor: Favre, Daniel, 1205 Genève (CH)
(74) Representative: Kiliaridis, Constantin

(57) **Abstract**

The invention related to a process for infecting eukaryotic cells with one or more virus species, preferentially hepatitis B or hepatitis C virus, as well as cell cultures infected by the same. To achieve this goal, plasma or serum obtained from individuals infected by viruses, preferentially hepadnaviridae and flaviviridae, is used as an inoculum to infect established eukaryotic cell lines or primary cells, preferentially hepatocytes, which in turn produce viral particles. This process, the resulting infected cells and cell culture supernatant can be used in many situations, notably the screening of drug candidates, the detection of antibodies, the generation of a diagnostic kit and the production of vaccines.

## Description

### Field of the Invention.

The present invention relates to a method for *in vitro* infection of eukaryotic cell lines or primary cells with viruses as well as to the resulting infected cell cultures.

To achieve this goal, plasma or serum obtained from individuals infected by viruses, preferentially hepadnaviridae and flaviviridae, is used as an inoculum to infect established eukaryotic cell lines or primary cells, preferentially hepatocytes, which in turn produce viral particles. This process and the resulting infected cells and cell culture supernatant can be used in many situations, notably the screening of drug candidates, the detection of antibodies, the generation of a diagnostic kit and the production of vaccines.

The following references are either cited in the text or relevant to the prior art:
Acs et al, Proc. Natl. Acad. Sci. USA 84: 4641-4644 (1987)
Agnello et al, Proc. Natl. Acad. Sci USA 96: 12766-12771 (1999)
Aoki et al, Virology 250: 140-150 (1998)
Bchini et al, J. Virol. 64: 3125-3032 (1990)
Bertolini et al Res. Virol. 144:281-285 (1993)
Bouffard et al, J. Inf. Diseases 166:1276-1280 (1992)
Boyer and Reuben, "Chronic hepatitis", chap. 23, pp. 586-637, Diseases of the liver, 7th edition, ( Schiff L and Schiff ER, eds), JB Lippincott Company, Philadelphia, 1993)
Burstein and Samaille, Clin. Chim. Acta 5: 609 (1960)
Chang et al, EMBO J. 6: 675-680 (1987)
Chirgwin et al, Biochemistry 18: 5294-5299 (1979)
Choi et al, DNA Cell Biol. 17: 951-956 (1998)
Cooper et al, "Lipoprotein metabolism", Hepatology: a textbook of liver disease, third edition, chapter 34, pp. 92-130 (Zakim D and Boyer TD, eds., WB Saunders Company, 1996)
Cribier et al., Arch. Virology 143: 375-379 (1998)
Dash et al, Am. J. Pathol. 151: 363-373 (1997)
Davis "Hepatitis C", Schiff's Diseases of the Liver, eight edition, chap. 30, pp. 793-836, (Schiff E, Sorrell M and Maddrey WC, eds, Lippincott-Raven Publishers, Philadelphia, 1999)
Diot et al, J. Med. Virol. 36: 93-100 (1992)
Ferri et al, Eur. J. Clin. Invest. 27: 711-718 (1997)
Finley et al, Clin. Chem. 24: 931-933 (1978)
Flint et al, J. Virol. 73: 6235-6244 (1999)
Forgac, "Receptor-mediated endocytosis", The Liver, Biology and Pathobiology, second edition, chapter 11, pp. 207-225, (Arias IM, Jakoby WB, Popper H, Schachter D and Shafritz DA, eds., Raven Press, Ltd, New-York, 1988).
Fredrickson et al, J. Clin. Invest. 47: 2446-2457 (1968)
Galle et al, Gastroenterology 106: 664-673 (1994)
Glickman and Sabesin, "Lipoprotein metabolism", The Liver, Biology and Pathobiology, second edition, chapter 18, pp. 331-354, (Arias IM, Jakoby WB, Popper H, Schachter D and Shafritz DA, eds., Raven Press, Ltd, New-York, 1988).
Gong et al, J. Viral Hepatitis 5: 377-387 (1998)
Gripon et al, J. Virol. 62: 4136-4143 (1988)
Gripon et al, Virology 192: 534-540 (1993)
Hayashi et al, "Hepatitis C virus and hepatocarcinogenesis", Intervirology 42: 205-210 (1999)
Hirschman et al, Proc. Natl. Acad. Sci. USA 77: 5507-5511 (1980)
Hollinger, "Hepatitis B virus", chap. 86, pp. 2739-2807, Fields Virology (Fields BN and Howley PM, eds.,Lippincott-Raven Publishers, Philadelphia, 1996)
Iacovacci et al, Res. Virol. 144: 275-279 (1993)
Iino "Problems in the treatment of hepatitis c with interferon" Intervirology 42: 166-172 (1999)
Jacob et al, Hepatology 10: 921-927 (1989)
Koff, "Viral hepatitis", chap. 21, pp. 492-577, Diseases of the liver (Schiff L and Schiff ER, eds, Lippincott Company, Philadelphia, 1993)
Lamas et al, J. Hepathol. 16:219-223 (1992)
Lanford et al, Virology 202: 606-614 (1992)
Lohmann et al, et al Science 285: 110-113 (1999)
Mabit et al, J. Gen. Virol. 75: 2681-2689 (1994)
Macpherson and Montagnier, Virology 23: 291-294 (1964)
Mehdi et al, J. Virol. 68: 2415-2424 (1994)
Monazahian et al, J. Med. Virol. 57: 223-229 (1999)
Moradpour et al, Hepatology 28: 192-201 (1998)
Nakajima et al, J. Virol. 70: 3325-3329 (1996)
Ochiya et al, Proc. Natl. Acad. Sci. USA 86: 1875-1879 (1989)
Parkin et al, "The global health burden of infection associated cancers,
Seef, "Diagnosis, therapy, and prognosis of viral hepatitis", p. 1067-1145, Hepatology: a textbook of liver disease, 3rd edition (Zakim D and Boyer TD, eds, WB Saunders Company, 1996)
Pileri et al, Science 282: 938-941 (1998)
Reid et al, "Extracellular matrix and hormonal regulation of synthesis and abundance of messenger RNAs in cultured liver cells", The Liver: Biology and Pathobiology, second edition, chap. 39, pp. 717-735 (Arias IM, Jakoby WB, Popper H, Schachter D and Shafritz DA, eds., Raven Press, Ltd, New-York, 1988)
Robinson, "Biology of human hepatitis viruses", Hepatology: a textbook of liver disease, third edition, chapter 37, pp. 1146-1206 (Zakim D and Boyer TD, eds., WB Saunders Company, 1996)
Rumin et al, J. Viral Hepatitis 3: 227-238 (1996)
Russel et al, Clin. Chem. 28: 1379-1388 (1982)
Schinazi et al, Antiviral Chem. Chemother. 10: 99-114 (1999)
Seipp et al, J. Gen. Virol. 78: 2467-2476 (1997)
Shibayama et al, J. Med. Virol. 13: 205-214 (1984)
Shimizu et al, Proc. Natl. Acad. Sci. USA 89:5477-5481(1992)
Shimizu et al, J. Virol. 71:5769-5773 (1997)
Steer, "Receptor-mediated endocytosis: mechanisms, biologic functions, and molecular properties" Hepatology: a textbook of liver disease, third edition, chapter 6, pp. 149-214 (Zakim D and Boyer TD, eds., WB Saunders Company, 1996)
Sureau et al, Cell 47: 37-47 (1986)
Sureau et al, Arch. Virol. [Suppl.] 8: 3-14 (1993)
Tagawa et al, J. Gastroenterol. Hepatol. 10: 523-527 (1995)
Thomssen et al. Med. Microbiol. Immunol. 181: 293-300 (1992)
Thomssen et al, Med. Microbiol. Immunol. 182: 329-334 (1993)
Treichel et al, Arch. Virol. 142: 493-498 (1997)
Tur-Caspa and Laub, J. Hepatol. 11: 34-36 (1990)
Weiss et al, Virology 216: 214-218 (1996)
WHO annual report 1996
Wild and Hall, "Hepatitis B virus and liver cancer: unanswered questions", Cold Spring Harbor Laboratory Press, Cancer Survey, vol. 33, pp. 35-54, Infections and Human Cancer, 1999 Imperial Cancer Reserach Fund
Yaginuma et al, Proc. Natl. Acad. Sci. USA 84: 2678-2682 (1987) Yoo. Et al, J. Virol. 69: 32-38 (1995)
Zignego et al, J. Hepathol. 15:382-386 (1992)
Zoulim and Trepo, Intervirology 42: 125-144 (1999)

### Background of the Invention.

Viral hepatitis is defined as "a systemic viral infection in which hepatic cell necrosis and hepatic inflammation lead to a characteristic constellation of clinical, biochemical, immunoserologic and morphologic features... caused by viral agents with distinctive immunoserologic characteristics and specific epidemiologic attributes" (Koff., 1993). The biology of hepatitis viruses and the description of the liver diseases due to these viruses have been reviewed extensively (Robinson, 1996; Koff, 1993; Seef, 1996). At least seven viral agents are involved in the viral hepatitis, namely, hepatitis viruses A (HAV), B (HBV) , C (HCV), D (HDV), E HEV), G HGV), and TT (TTV). All these viruses, except for HAV and HEV, cause chronic hepatitis. For the purpose of the present invention, the term "virus" encompasses both wild-type and mutant strains.

Infections with hepatitis viruses are the most prevalent viral diseases in the world. Hepatitis B virus (HBV - a member of hepadnaviridae family) and hepatitis C virus alone (HCV - a member of flaviviridae family) affect respectively 350 million and 100 million individuals worldwide (WHO annual report, 1996). These viruses are known to cause both acute and chronic liver disorders. In addition, epidemiological studies have linked HBV and HCV infections with the formation of hepatocellular carcinoma (HCC) (Parkin, 1999, pp. 5-11; Wild and Hall, 1999, pp. 35-54; Ferri, 1997; Boyer and Reuben, 1993, p. 611; Hayashi, 1999, pp. 205-210). Current treatments of HBV- and HCV-infected individuals include interferon and antiviral drugs. However, their efficiency is not fully convincing and they are not devoid of significant side effects.

In order to study hepatitis virus-associated diseases and develop new treatments, one needs a reliable means for cultivating hepatitis viruses. To date, only chimpanzees, mice, rats, tree shrews, woodchucks and ducks can serve as models for the study of the hepatitis B and C infections (Schinazi, 1999). Such animal models have many disadvantages such as high costs, complexity of observations, unsuitability to high-throughput screening for new therapeutic agents, and significant animal suffering. During the recent years, several attempts have been made in order to produce HBV or HCV in eukaryotic cells *in vitro.* For the purpose of the present invention, *"in vitro* viral propagation" and equivalent wordings mean the replicative production in cell culture of fully intact infectious virus, e.g. human hepatitis virus.

Two main procedures for *in vitro* propagation may be used:
i) the infection of cells with a viral inoculum.
   "Infection" is defined as follows: the agency, substance, germ or principle by which an infectious disease is communicated or transmitted; the action or process of infecting.
ii) the transfection of cells with nucleic acids. "Transfection" is defined as follows: introduction of foreign genetic material into cells.

The current "state-of-the-art" model for the replication of HBV and HCV in tissue culture is essentially based on the transfection - and not the infection - by genetic materials from HBV, respectively HCV of established hepatocyte cell lines, such as the HepG2 (ATCC HB8065) and the Huh-7 cell lines (obtained from Dr. Helmut Jacobsen, F. Hoffmann-La-Roche, Pharma Division, Preclinical Central Nervous System Research, Basel, Switzerland.) (Tables 1 and 2 at the end of the description).

In one prior art experiment, the HepG2 cell line was transfected with a DNA clone containing two tandemly arranged copies of the HBV genome. The resulting cell line ("2.2.15 cells") produces HBV surface antigen, nucleocapsids and virions. However, the 2.2.15 cell line has several disadvantages:
1) the rate of production of infectious particles (Dane particles) by the 2.2.15 cell line is very low.
2) the cell line produces continuously an important amount of replicative intermediates, which must first be washed out of the involved cells before testing for the virus titer in experiments with antiviral compounds.

For these reasons, initially transfected cell lines such as HepG2 and Huh-7 (which has similar features) are not suitable for large-scale or high-throughput screening.

Many different receptors have been mentioned in prior art research concerning the entry of hepatitis B and hepatitis C virus particles into cells during the infection process.
Concerning HBV, the albumin receptor, the transferrin receptor, the IgA receptor, the low density lipoprotein (LDL) receptor, the asialoglycoprotein (ASPBG) receptor, the interleukin-6 receptor, the endonexin-2 receptor, the GAPD receptor, and putative receptors of 31 kilodaltons (kDa) and 50 kDa, as reviewed by Treichel (Treichel, 1997; and references therein) have all been mentioned in literature. However, there is no definitive answer as to which of these receptors is directly responsible for the entry of HBV particles in the cells.

Concerning HCV, the CD81 receptor (Pileri, 1998; Flint, 1999), the LDL receptor (Agnello, 1999), the mannose receptor and the asialoglycoprotein receptors (Houghton et al., U.S. Patent No. 5,968,775) have all been mentioned in literature as a possible mechanism of entry. However, there is no definitive answer as to which of these receptors is directly responsible for the entry of HCV particles in the cells.

Notably, a recent work by Cribier et al. (1998) indicates that pretreatment by dextran sulfate of the cells to be infected by HCV inhibits the attachment of HCV to the cell surface and prevents any infection of the cells. This finding suggests that by removing lipoproteins or lipids, dextran sulfate inhibits the interaction between the virus particles and the cells. Accordingly, the prior art specifically teaches away from using dextran sulfate or similar compounds in *in vitro* infection systems, especially for hepatitis viruses.

To summarize, despite many attempts, no efficient and reliable *in vitro* infection system for HBV and HCV has been described in the prior art. (Robinson, 1996, pp.1149, 1155, 1161; Koff, 1993, pp. 498-499; Hollinger, 1996, p. 2742; Ganem, 1996, p. 2709; Robinson, 1996, p. 1189; Lohmann et al, 1999, reference 6 therein; Schinazi, 1999).

The characteristics required for a reliable and efficient in *vitro* cell culture system have been described for the culture of hepatitis B virus (Sureau, 1993). These characteristics, which also apply for the hepatitis C virus, are the following:
i) the cells should be of human origin.
ii) they should be immortalized in order to ensure an unlimited supply.
iii) the cells should retain the characteristics and functions of differentiated hepatocytes.
iv) they should be both susceptible to and permissive for HBV, respectively HCV.
v) they should sustain replication in quantities sufficient for most biochemical and molecular analysis procedures.

Accordingly, there is an important need for an HBV and HCV infection system satisfying these characteristics.

### Brief description of the Invention.

The solution to the above technical problem is achieved by providing the embodiments characterized in the claims. The present invention provides a reliable and efficient infection system for eukaryotic cells satisfying the characteristics just described for HBV, HBC, and other viruses.

The goal of infecting eukaryotic cells with one or more virus species is attained by freeing the receptors for lipoproteins and/or lipids at the cell surface from essentially all lipoproteins and/or lipids prior to infection by the contemplated virus. It is understood that the absence of lipoproteins and/or lipids on these receptors allows proper interaction between the virus particles (themselves covered with lipoproteins) and the targeted cells.

The present invention can be used for infection with any virus species, preferentially hepatitis-causative agents, such as hepadnaviridae (HBV and others), flaviviridae (HCV, dengue, yellow fever and others), or any combination of virus species, such as hepatitis delta with hepatitis B virus.

The cells to be infected according to the present invention can be any eukaryotic cells, be they primary or from an established cell line. Preferentially, the cells are from established hepatocyte cell lines such as HepG2 or Huh7, hepatocyte primary cells, any eukaryotic cell of primate origin as well as any eukaryotic cell expressing a lipoprotein and/or lipid receptor. It is understood that said expression occurs either naturally or after cloning of the suitable gene in the eukaryotic cell.

The agent used for freeing the receptors for lipoproteins and/or lipids at the cell surface from essentially all lipoproteins and/or lipids prior to infection by the contemplated virus can be sulfated polysaccharides such as dextran sulfate or heparin compounds, or chelating agents such as ethylene glycol-bis (beta-aminoethyl ether) N,N, N', N'-tetraacetic acid (EGTA; ethylene-bis(oxyethylenenitrilo) tetraacetic acid; egtazic acid) or ethylenediaminetetraacetic acid (EDTA; [ethylenedinitrilo] tetraacetic acid), or any other agent capable of removing the cell-bound lipoproteins and/or lipids from the surface receptors.

The present invention also relates to the infected cell cultures obtained by performing the claimed process. When compared to prior art transfected cell lines (such as HepG2 and Huh-7), the infected cell cultures of the present invention achieve a much higher rate of production of infectious particles, and do not product a significative amount of replicative intermediates.

Accordingly, unlike prior art cultures, the infected cell cultures of the present invention are suitable to large-scale or high-throughput *in vitro* screening of antiviral agents against infecting viruses. The procedure for screening antiviral compounds comprises at least one control sample and at least one test sample; each sample comprizes a substantially similar or equivalent amount of cells from the culture cell line, in this respect in a substantially similar or equivalent volume of cell culture fluid. Furthermore, before, during or after the infection process described in this invention, the test sample is exposed to potentially effective anti-HCV or anti-HBV agent(s) in a nutrient cell culture medium or any convenient fluid. The cells from the control sample and the cells from the test sample are thereafter incubated in convenient cell culture medium during a convenient incubation time. Products, comprising expressed ribonucleic acids (in the case of both HBV and HCV), expressed deoxyribonucleic acids (in the case of HBV), or produced polypeptides are then detected in both the control sample and in the test sample by employing conventional detection methods. The antiviral properties of the tested compound can be rated according to the amount of products produced. No of few products mean that the tested compound has antiviral properties, and conversely.

In addition, the infected cell cultures of the present invention also provide a means to produce antigen-containing medium. A new and important clinical diagnosis and prognosis tool is thus provided by the above-mentioned viral antigens, which can be used in the detection of antibodies against HBV, HCV and other viruses in the sera of the patients. This can be achieved by mixing the serum of a patient with the viral antigens that are produced by the aforementioned cells, and then by screening for the antigen-antibody interactions using conventional methods.

Finally, the viral antigens produced by the infected cell cultures of the present invention can be used for the generation of a vaccine. Existing vaccine preparations against HBV (Recombivax HB and Engerix-B; see Seef, 1996, p. 1119; Hollinger, 1996, p. 2787) have variable immunological performances, due to the fact that antigens produced through recombinant technology are incomplete and may lack immunologically important epitopes. Such epitopes can be preserved in the *in vitro* infection system of the present invention. Infectivity of the virus particles produced in said system can be attenuated or inactivated by conventional methods, such, for example, treatment with urea, pepsin, formaldehyde, or exposure to ultraviolet light.

Other advantages of the present invention include the low cost incurred in obtaining infected cultures and the fact that living animals such as chimpanzees are no longer needed as a model for infection.

The invention is further described in the following examples which set forth particularly advantageous embodiments. These embodiments are indeed intended to be exemplary and illustrative, but not limiting, in the sense that they are not restricting the present Invention in any way.

### Legends to figures.

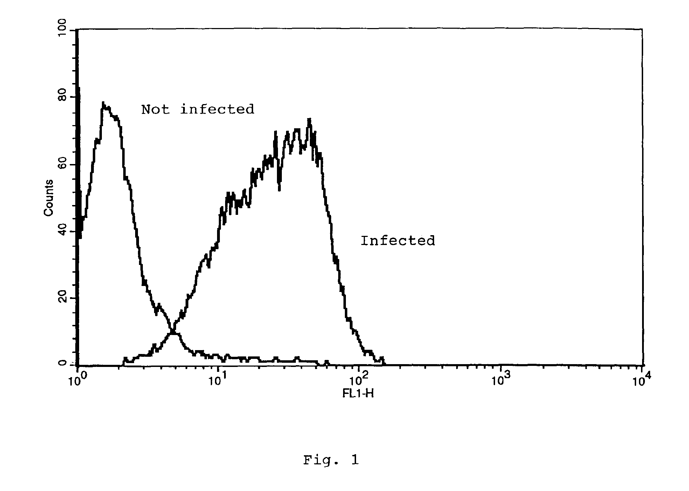

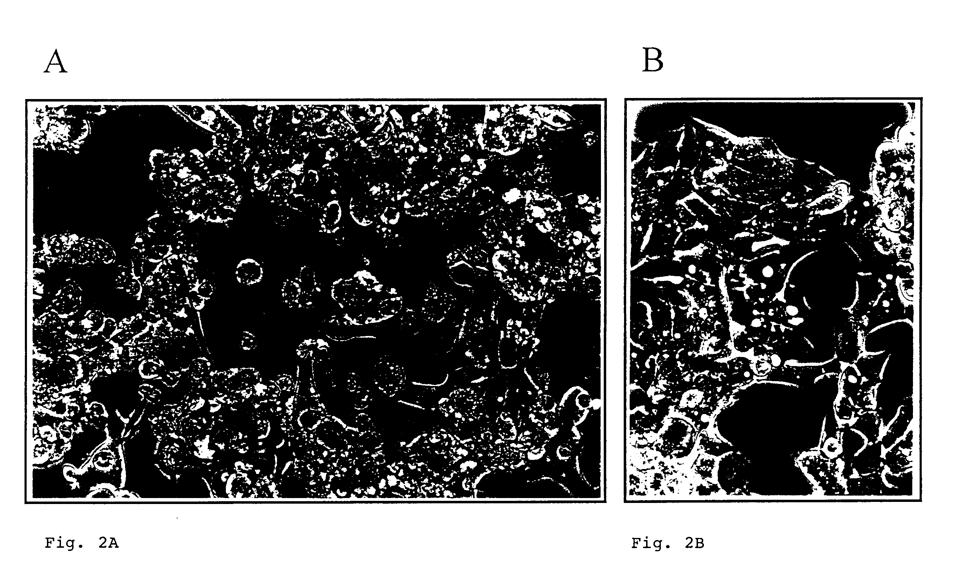

Figure 1:Percentage of HBV-infected cells expressing HBsAg at their cell surface.

Figures 2A to 2D :

HBV-infected HepG2 cells in monolayers. (A) and (C): HBV-infected HepG2 cells 18 days post-infection; (A) incubation in complete medium, (C) in a serum-free defined medium. (B) and (D): mock-infected HepG2 cells 18 days post mock-infection; (B) in complete medium; (D) in serum-free defined medium. Magnification: 2000x

Figures 3A and 3B

HBV-infected HepG2 cells as suspensions in soft agar. HepG2 cells were infected with HBV for 10 days, trypsinized, washed, and then seeded into complete medium containing soft agar. Large clumps of cells are growing in the soft agar after three weeks of incubation. Cells in suspension were estimated to divide every two to three days. Dark cells are probably necrosed cells. Magnification: (A) 500x and (B) 2000x. Non-infected cells and mock-infected cells do not grow in soft agar (not shown).

Figures 4A and 4B:

Immunofluorescence studies of HBV-infected HepG2 cells. (A) In this experiment, around 68% of the cells are stained with the FITC-labeled antibody directed against HBsAg 12 days post-infection. (B) phase contrast microscopy of the same field. Magnification (A) and (B): 2000x.

Figures 5A to 5E

Growth of HBV-infected HepG2 cells in hormone-supplemented complete medium: effect of insulin plus dexamethasone. Infected cells were grown in complete medium for two weeks, trypsinized, washed, and thereafter grown in complete medium supplemented with insulin plus dexamethasone. Througout the incubation in complete medium containing insulin plus dexamethasone that was changed every three to four days, general features do emerge: (A) and (B) aberrant morphologies of attached, HBV-infected cells, and appearance of rounded cells on the cell culture flask substrate; (C) rounded cells on the cell culture flask substrate do divide every two to three days, starting to produce clumps; (D) after around four weeks, large clumps containing thousands of cells have formed; (E), as (D), with higher magnification. Magnification: (A), (B) and (E) 2000x; (C) 500x; (D) 800x

### Detailed description of the invention

### 1) Culture medium.

Although each type of primary cell or cell line usually grows better on a specific culture medium, other cell culture formulations may be used for the supportive growth of the considered cells. In order to delineate the field of the concerned cell culture protocols and media formulations, the skilled persons might take the relevant literature into consideration, such as for example: Freshney RI, "Culture of animal cells: a manual of basic techniques" (3rd edn.), Wiley-Liss, Inc (1994); "Mammalian Cell Culture: essential techniques", (Doyle A & Griffiths JB, eds.), John Wiley & sons (1997). Since hepatocytes are taken into consideration, cells can also be grown in improved formulations consisting in serum-free, hormonally-defined media, as described by Reid (Reid, 1988).

### 2) Cells.

HepG2 cells (ATCC HB8065) were grown in Eagle's Minimal Essential medium (EMEM; Biochrom AG, Berlin, Germany) supplemented with 10% fetal calf serum (FCS; PAA, Linz, Austria), 2 mM L-glutamine (Gibco-Life technologies, Paysley, Scotland) and Earle's BSS (Gibco), and adjusted to contain 1% non-essential amino acids (Amimed Bioconcept, Allschwil, Swizerland), 1 mM sodium pyruvate (Gibco), 25 mM HEPES (Gibco), 1.5 g/l sodium bicarbonate (Amimed), and antibiotics (100 U/ml penicillin, 100 micrograms/ml streptomycin; Gibco) (complete medium). After washing in phosphate buffered saline (PBS; per liter: NaCl 8 g/l, KCl 0.2 g/l, KH2P04 0.2 g/l, Na2HPO4(2H2O) 1.44 g/l) and trypsinization (Trypsin-EDTA; Gibco), the cells were either directly seeded at 25 to 50% confluency into new culture flasks, or the cell monolayer was trypsinized as above and the cells were washed thereafter once in complete medium before seeding into new culture flasks. This supplementary wash after trypsinization is optional according to the proposed protocol for the trypsinization of the HepG2 cells, but it revealed that the cells were growing as a true monolayer after their seeding into the new culture flasks. Cultures were observed using a phase-contrast microscope. This revealed that the doubling time was around 8 to 10 days in the conditions employed. The totality of the cell culture medium was changed every 2 to 3 days.

Huh-7 cells were grown in Iscove's Modified Eagle Medium (IMEM; Gibco) supplemented with 10% fetal calf serum, 1% sodium pyruvate, 2 mM L-glutamine and antibiotics (100 U/ml penicillin, 100 micrograms/ml streptomycin) (complete medium).

If other relevant cell lines or primary cells are employed which would grow preferably as suspension cultures, the trypsinization step described above can be omitted with such cells.

### 3) Infection of cell cultures.

The cells are grown in complete medium as described above. For infection, the following procedure is employed.
1. The cells are first optionally washed with PBS.
2. Following this optional wash, the cells are treated with dextran sulfate, for example 10 milligrams per milliliter in convenient fluid, during an incubation period of 5 to 10 minutes, for example. Alternatively, heparin, ethylene glycol-bis (beta-aminoethyl ether) N,N, N', N'-tetraacetic acid (EGTA; ethylene-bis(oxyethylenenitrilo) tetraacetic acid; egtazic acid), ethylenediaminetetraacetic acid (EDTA; [ethylenedinitrilo] tetraacetic acid) might be employed instead of dextran sulfate. The incubation with dextran sulfate or said other agents allows the freeing of the surface receptors for lipoproteins and/or lipids from essentially all lipoproteins and/or lipids prior to infection.
3. After the incubation of the cells with dextran sulfate or said other agents, the cells are extensively washed with PBS to remove traces of dextran sulfate.
4. The viral inoculum is then added to the cells for an incubation period of 30-90 minutes. This viral inoculum might consist in either i) undiluted plasma, or serum, said plasma or serum containing relevant titers or relevant genome equivalents of virus, said virus belonging to the family hepadnaviridae or flaviviridae (such as hepatitis B virus or hepatitis C virus, respectively) or any other virus species; or ii) serum or plasma diluted in convenient fluid, said fluid having various physiologically relevant formulations. Alternatively, the viral inoculum can originate from another source than blood, such as an other in vitro cell culture in which the cells have been infected in a previous experimental process, said cells being an established primary culture or cell line, or from the resuspension of ultracentrifuged virus or immunoprecipitated virus, or from blood obtained from an infected chimpanzee.
5. After the infection, the viral inoculum is removed from the cells, and the cells are extensively washed with PBS. The cells are then incubated in complete medium. Optionnally, the complete medium can be changed after an over-night incubation in order to remove unbound input virus, and thereafter the cells are put with new complete medium.

Alternatively, a higher rate of infection is achieved if one or more steps of the infection process described above is performed at lower temperatures, for example at 4 degrees Centigrade or on ice. In this respect, the cells in complete medium can first be placed on ice for an incubation period of about 10 minutes. The cells are then optionally washed with ice-cold PBS. The cells are then treated on ice with dextran sulfate, as described above. After the removal of the dextran sulfate, the cells are washed extensively with ice-cold PBS, and the ice-cold viral inoculum is added to the cells, as described above. The cells are then incubated with the viral inoculum by placing them directly at 37 degrees Centigrade for 1 h as described above, or the cells can be kept for a while on ice, said for example 10 minutes, before being kept at 37 degrees Centigrade. After the incubation of the cells with the viral inoculum, the viral inoculum is removed, the cells are washed as descibed above, and the cells are incubated with complete medium as descibed above. Optionnally, the complete medium can be resplenished after an over-night incubation, as described above.

### 4) Maintenance of infected cell cultures.

As described above, the complete medium from uninfected cell cultures is normally changed every two to three days. However, during infection experiments lasting up to a week, the same culture medium was kept during the entire course of the infection. In long term infections lasting longer, the culture supernatant was periodically resplenished with complete medium, for example every 7 to 10 days, in order to avoid significative evaporation of the supernatant.

### Example 1

This example demonstrate the effective and sustainable infection of the HepG2 cell line by HBV. Proof of infection by HBV is supported by the following tests: presence of HBsAG in supernatant (a) and associated with cells (b), (c), and (d); transformation effect of HBV (e); excess growth with combined treatment with dexamethasone-insulin (f); long term incubation (g).

### (a) Presence of HBsAg in the cell culture supernatant.

For the detection of HBsAG either in the supernatant or at the cell surface, the AXsYM (Abbott Laboratories) routine test was commonly employed for the detection of the HBV surface antigen. To keep a convenient concentration of proteins in the samples in order to get accurate results, the following procedure was usually taken into consideration: the cell culture supernatants from mock-infected and HBV- infected cultures were usually mixed with AUSAB-negative, HBV-negative human serum, usually to get 50% to 90% of serum, final concentration, in the samples to be analyzed. The results are given in the form of an index: index values below 2.1 reveal the absence of HBsAg in the analyzed sample. Index values over 2.1 reveal the presence of HBsAg in the analyzed sample. Some parallel control analysis revealed that for research purposes, the results can also be accurately obtained without the addition of AUSAB-negative, HBV-negative human serum in the samples to be analyzed: aliquots of HBV-positive sera can also be mixed with other fluids such as fetal calf serum in order to keep convenient concentrations of proteins in the samples, and the results obtained with the test are similar. The AXsYM routine test (Abbott laboratories)was used on: i) mock-infected cells; ii) HBV-infected cells that were infected by following the process described in this invention. Please refer to Table IIIa for results.

### (b) Presence of HBsAg associated with cells.

Around 2 X 10 sups HepG2 (representing approximatively 20 microliters of cytoplasm equivalent) were suspended in 500 microliters serum, frozen/thawed three times, centrifuged, and the resulting supernatant was diluted 100-fold for analysis of HBsAg on AXsYM. Results are expressed as absolute S/N ratios, the original S/N ratio from the considered measure being multiplied by the corresponding dilution factors. The results reveal that the majority of the HBsAg is cell-associated.

### (c) percentage of HBV-infected cells expressing HBsAg at their cell surface as determined by IF.

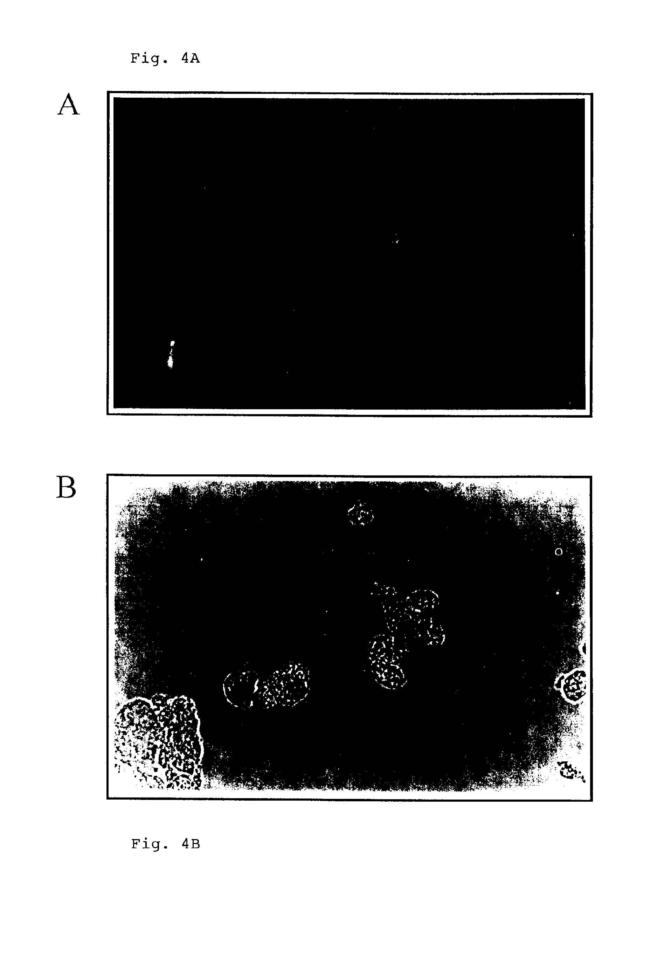

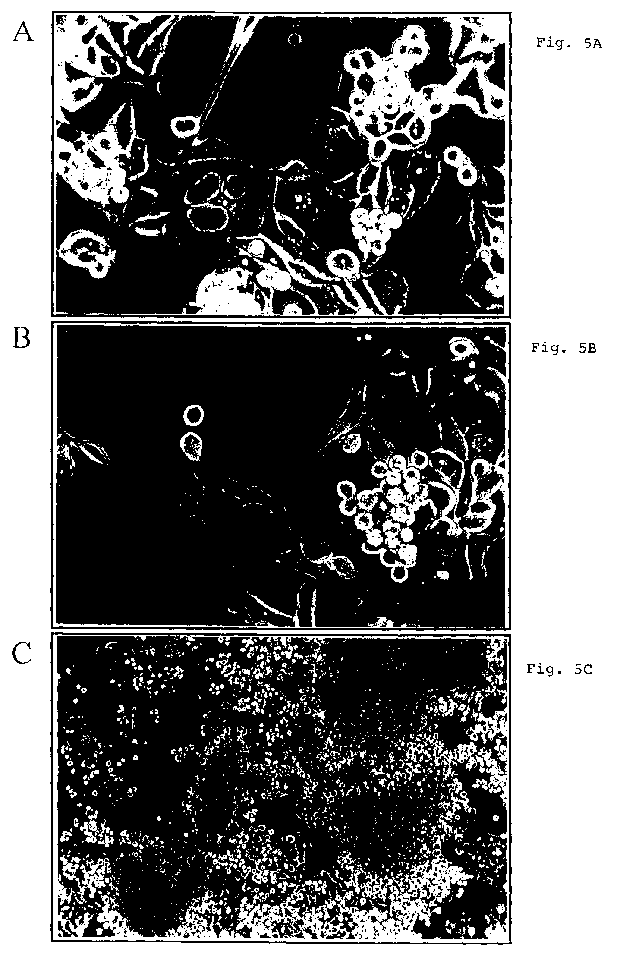

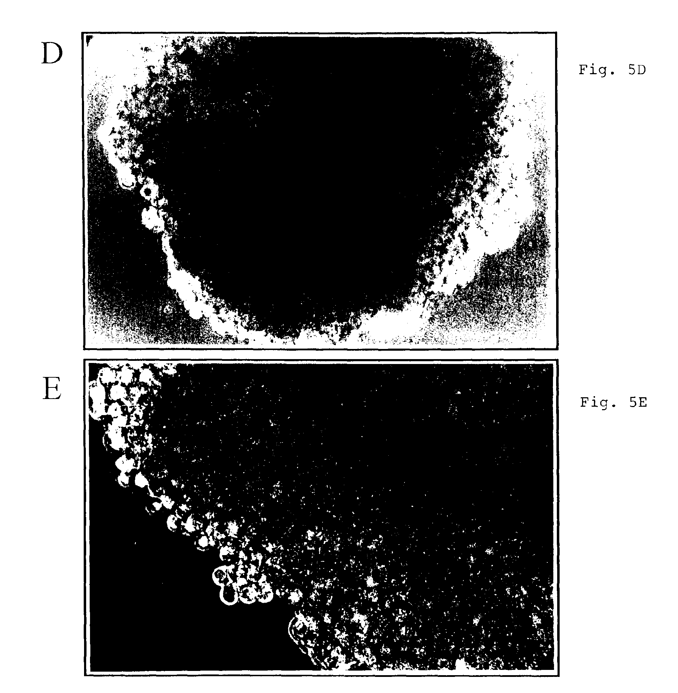

For the determination of the percentage of cells associated with HBsAG, the following protocol was used. Infected HepG2 cells (around 10 sup 6) were trypsinized and washed in complete medium. The cells were then resuspended in 80 microliters of phosphate buffered saline (PBS) containing 0.2% fetal calf serum (FCS) and fluorescein isothiocyanate (FITC)-labeled, goat anti-hepatitis B surface antigen (Anawa Trading, Wangen Zurich, Switzerland; cat. No 4940-1464) at a 1/50 titer. The cells were then incubated for 1 hour on ice, followed by two or three washes with ice-cold PBS. The cells were then fixed in 500 microliters of PBS containing 1 to 2% paraformaldehyde, or acetone. Uninfected cells were used as negative control. Positive controls have been established by the original supplier of the above-mentioned FITC-labeled, anti-HBsAg antibody by employing an hepatocyte cell line that is constitutively expressing the HBsAg. The cells were observed with a microscope equipped with epifluorescence (Carl Zeiss, Axiophot 35, Oberkochen, Germany). Immunofluorescence studies of HBV-infected cells by using a FITC-labeled antibody directed against HBsAg. Figure 4.

### (d) percentage of HBV-infected cells expressing HBsAg at their cell surface as determined by FACS.

In addition to the protocol above, the cells processed for immunofluorescence as described above were also analyzed by fluorescence-associated cell sorting (FACS; FACSCalibur, Becton Dickinson).Cf Figure 1.

### (e)transforming effect of HBV

For the determination of the transforming effect of HBV, the so-called soft-agar assay was used. It is known for more than three decades that an agar suspension culture is a selective assay for the transformation of cells with a virus (Macpherson I. and Montagnier 1., 1964). This assay is called "clonogenic assay", and relevant protocols have been established, for example protocol 17 "a soft agar clonogenic assay for anchorage-dependent cells" in "Mammalian Cell Culture: essential techniques", (Doyle A & Griffiths JB, eds.), John Wiley & sons (1997). Usually, and according to the literature in the field and not solely to this proposed protocol, the agar concentrations can range between 0.2 to 0.8%, final concentration. The HepG2 cell line does not grow in soft agar. Thus, if this cell line is infected with a virus, the latter being able to transform the cells, these cells might grow in soft agar. In order to determine, whether HBV is a transforming virus, the HepG2 cells have been infected as described above, incubated in a first time as monolayers for around two to three weeks in complete medium, trypsinized, washed in complete medium, processed for the soft agar assay as described above, however, in presence of 0.8% soft agar (Noble Agar, Difco; Detroit, USA) in complete medium, and finally incubated for a three week incubation period.

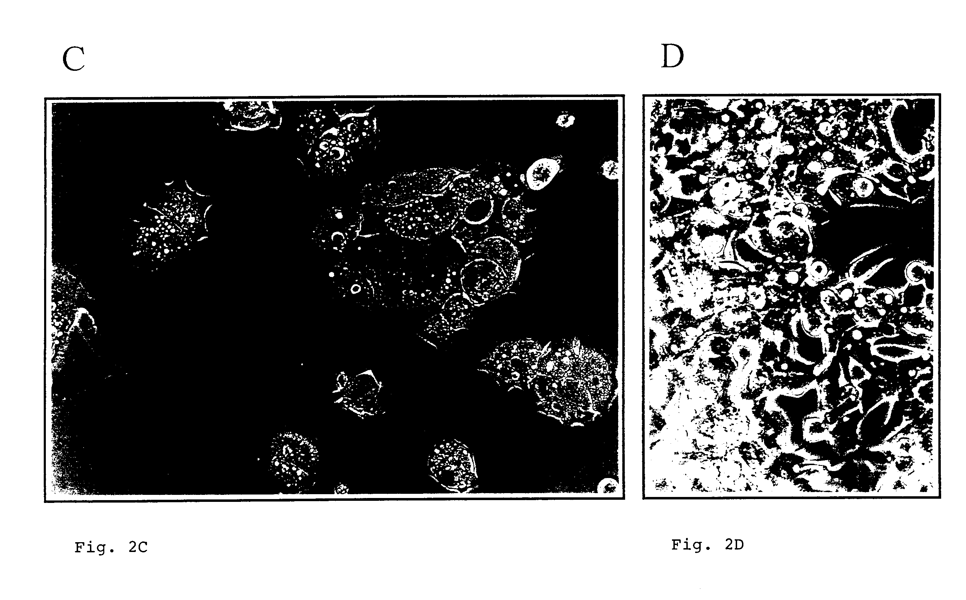

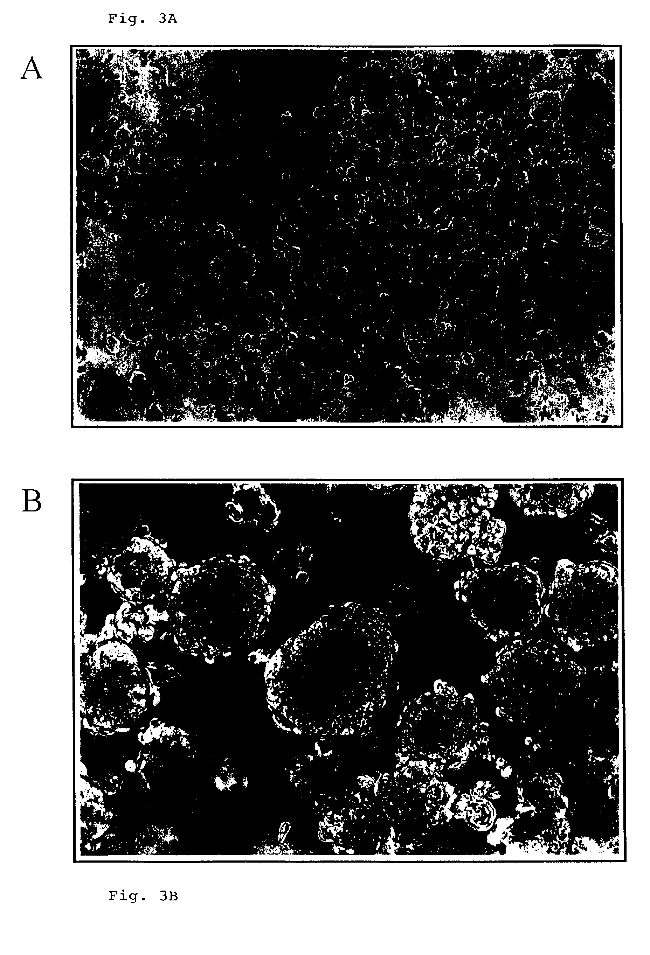

Soft agar assay of HBV-infected cells. Figure 3.

### (f) effect of additional insulin plus dexamethasone

Effect of additional insulin plus dexamethasone, in the complete medium, on HBV-infected cells. Figure 4.
This example is given to provide additional features in respect to the fate of the HBV-infected cells. Indeed, some relevant literature does already mention that insulin and glucocorticoids might play an important role when HBV is taken into consideration (Tur-Caspa and Laub, 1990; Choi, 1998). Thus, cells were incubated in complete medium containing around 10 sup minus 6 M of both insulin and dexamethasone. The complete medium containing insulin and dexamethasone was totally changed every 3-4 days.

### (g) Long-term incubation of HBV-infected cells. Table IV.

HepG2 cells were infected with a low titer of HBV, according to the method described in this Invention. The cells were then kept as monolayers for up to 35 days, and up to 38 days, respectively. During the whole incubation, solely a portion of around 75-80% of the volume of the complete medium was changed every five to seven days.

The cells (20 X 10 sup3) were analyzed by FACS as described above, at regular intervals.

**Table IV**

| Day after infection | % of HBsAg positive cells (HepG2) |
|---|---|
| 14 days | 14-16% |
| 21 days | 20-42% |
| 28 days | 43-58% |
| 38 days | 44-59% |

notes and observations: experiments performed in 12-wells plates (Costar); in the 10 surrounding wells in the considered plates, the percentage of HBsAg positive cells was always superior to the percentage of HBsAg positive cells growing in the two internal wells, thus explaining interindividual variations for the percentages measured.

### Example 2

This example demonstrate the effective and sustainable infection of the Huh7 cell line by HBV. Proof of infection by HBV is supported by long term incubation.
Huh-7 cells were infected with a low titer of HBV, according to the method described in this Invention. The cells were then kept as monolayers for up to 35 days (Table IVa). During the whole incubation, solely a portion of around 75-80% of the volume of the complete medium was changed every five to seven days.

The cells (20 X 10 sup3) were analyzed by FACS as described above, at regular intervals.

**Table IVa**

| Day after infection | % of HBsAg positive cells (Huh-7) |
|---|---|
| 11 days | 4-6% |
| 18 days | 22-29% |
| 25 days | 55-62% |
| 35 days | up to 82% |

notes and observations: experiments performed in 12-wells plates (Costar); in the 10 surrounding wells in the considered plates, the percentage of HBsAg positive cells was always superior to the percentage of HBsAg positive cells growing in the two internal wells, thus explaining interindividual variations for the percentages measured.

### Example 3

This example demonstrates the effective and sustainable infection of the HepG2 cell line by HCV. Proof of infection by HBV is supported by the detection of RNA in supernatant (COBAS Amplicor) and cytopathic effect.

For the detection of HCV, the COBAS Amplicor HCV Monitor test from Roche was routinely employed. To keep a convenient concentration of proteins in the samples in order to get accurate results, the following procedure was taken into consideration: the cell culture supernatants from mock-infected and HCV- infected cultures were usually mixed with HCV-negative human serum, usually to get 50% to 90% of serum, final concentration, in the samples to be analyzed. Negative controls were performed with HCV-negative sera; for the positive controls, human plasma or sera from patients infected with HCV with known HCV viremia were processed in a similar way than the samples from the culture medium obtained from mock- and HCV-infected cells.

The results are obtained in the form of an index: values below 0.2 do reveal the absence of viral RNA in the sample that is analyzed, and an internal control is provided with the analysis in order to ascertain that the result obtained is accurate. Index values over 0.2 do reveal the presence of viral RNA in the sample that is analyzed; usually, indexes obtained from HCV positive sera can reach an index value of around 3.5 to 3.8, and the analysis can be set up in order to provide an index of "9.999" when there is saturation of the sample with viral RNA.

Some parallel control analyses revealed that for research purposes, the results can also be accurately obtained without the addition of serum in the samples to be analyzed, since the method of extraction employed in the test does comprise extraction of the viral nucleic acids in 68% guanidine thiocyanate, 3% dithiotreitol, a features that is highly efficient for the extraction of ribonucleic acids in a whole range of biological samples (Chirgwin, 1979)

Analyses of the HCV RNA, by employing the Amplicor HCV Monitor test (Roche), in the cell culture supernatants of i) mock-infected cells, ii) cells infected with conventional methods, and iii) cells infected with the procedure described in this Invention. Table VIa (infection with various inoculum representing several genotypes of HCV, such as, for example, genotypes la, lb, 3 and 4). Table VIb (example of a typical infection using various dilutions of the same viral inoculum).

**Table VIa**

| | Mock-infected cells | Cells infected by using published protocols | Cells infected by following the procedure described in this Invention |
|---|---|---|---|
| Indexes COBAS Amplicor HCV Monitor: | Under 0.2 | Up to around 1.5 | Up to 3.8 (even "9.999") |

Inoculums containing various serums having various individual HCV genotypes with original RNA titers ranging from around 10 sup 6 to 56 X 10 sup 6 genomes per mililiter.

**Table vIb**

| | Indexes COBAS Amplicor HCV Monitor Cells infected by using published protocols | Indexes COBAS Amplicor HCV Monitor Cells infected by following the procedure described in this Invention |
|---|---|---|
| Inoculum with 50 microliters of HCV-containing serum | 0.2 a 1.5 | 3.770 |
| Inoculum with 5 microliters of HCV-containing serum | 0.2 a 1.5 | 3.594 |
| Inoculum with 0.5 microliters of HCV-containing serum | N.D. | 0.919 |
| Inoculum with 0.05 microliters of HCV-containing serum | N.D. | 0.035 |

Inoculum prepared with original serum having 56 X 10 sup 6 RNA genomes per mililiter (Roche). N.D.: not determined.

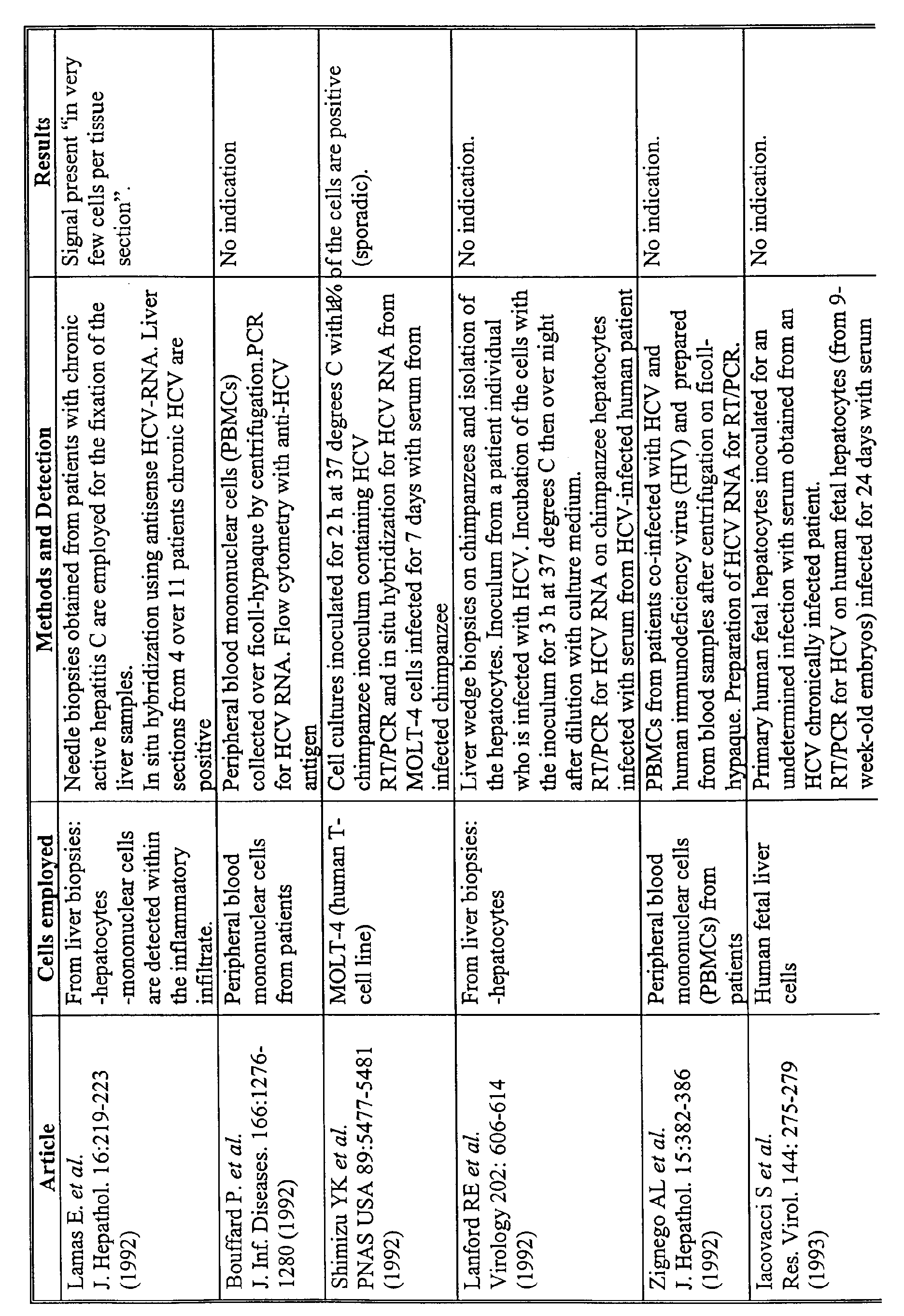

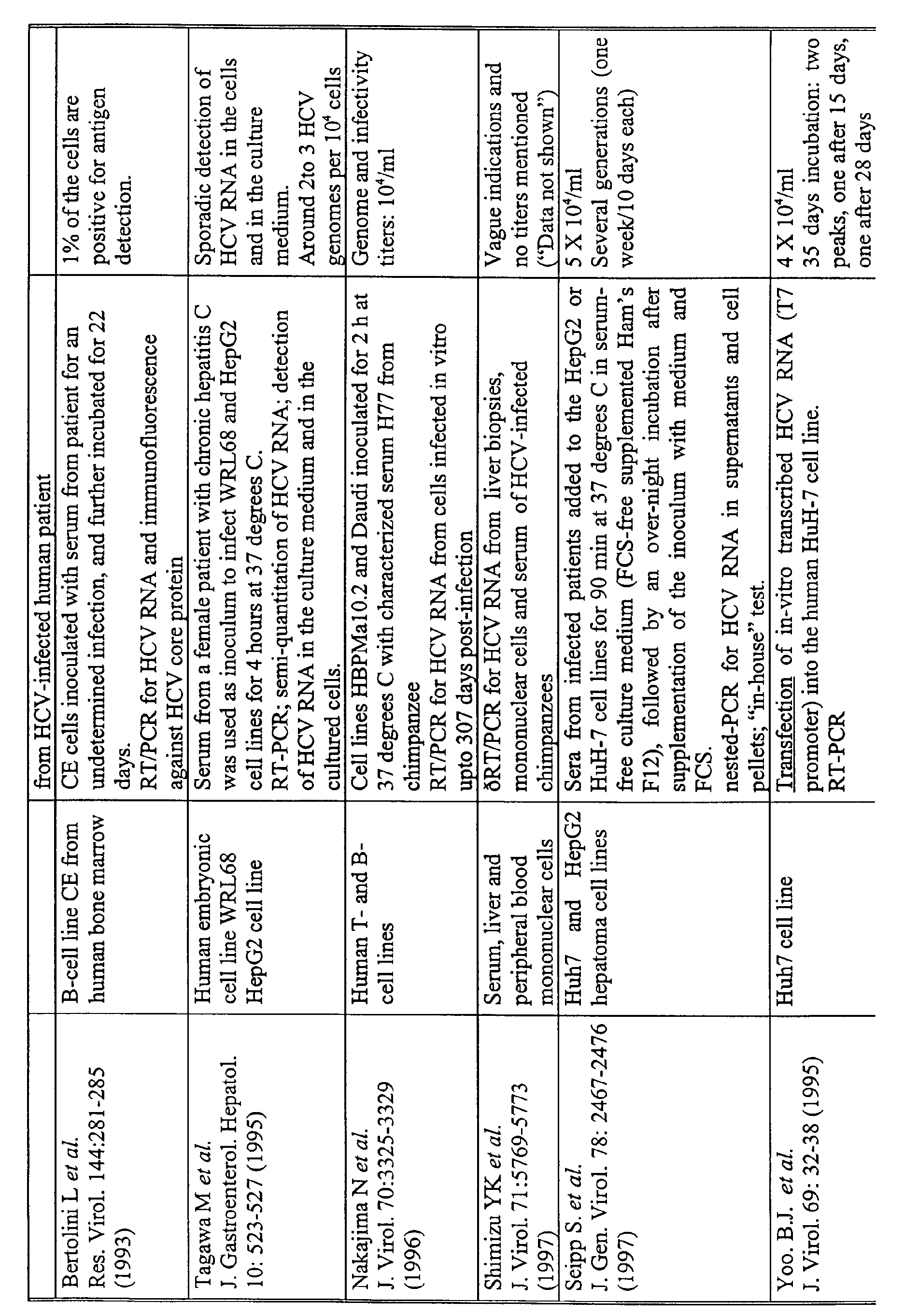

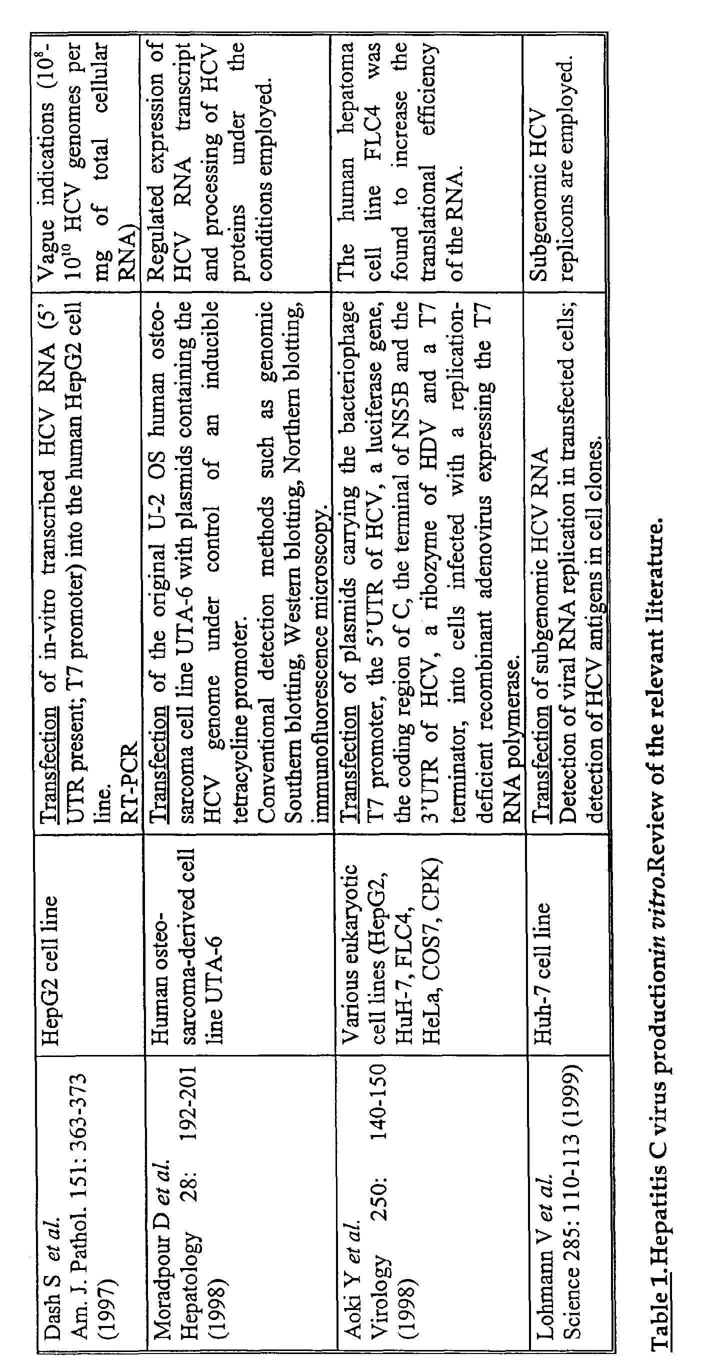

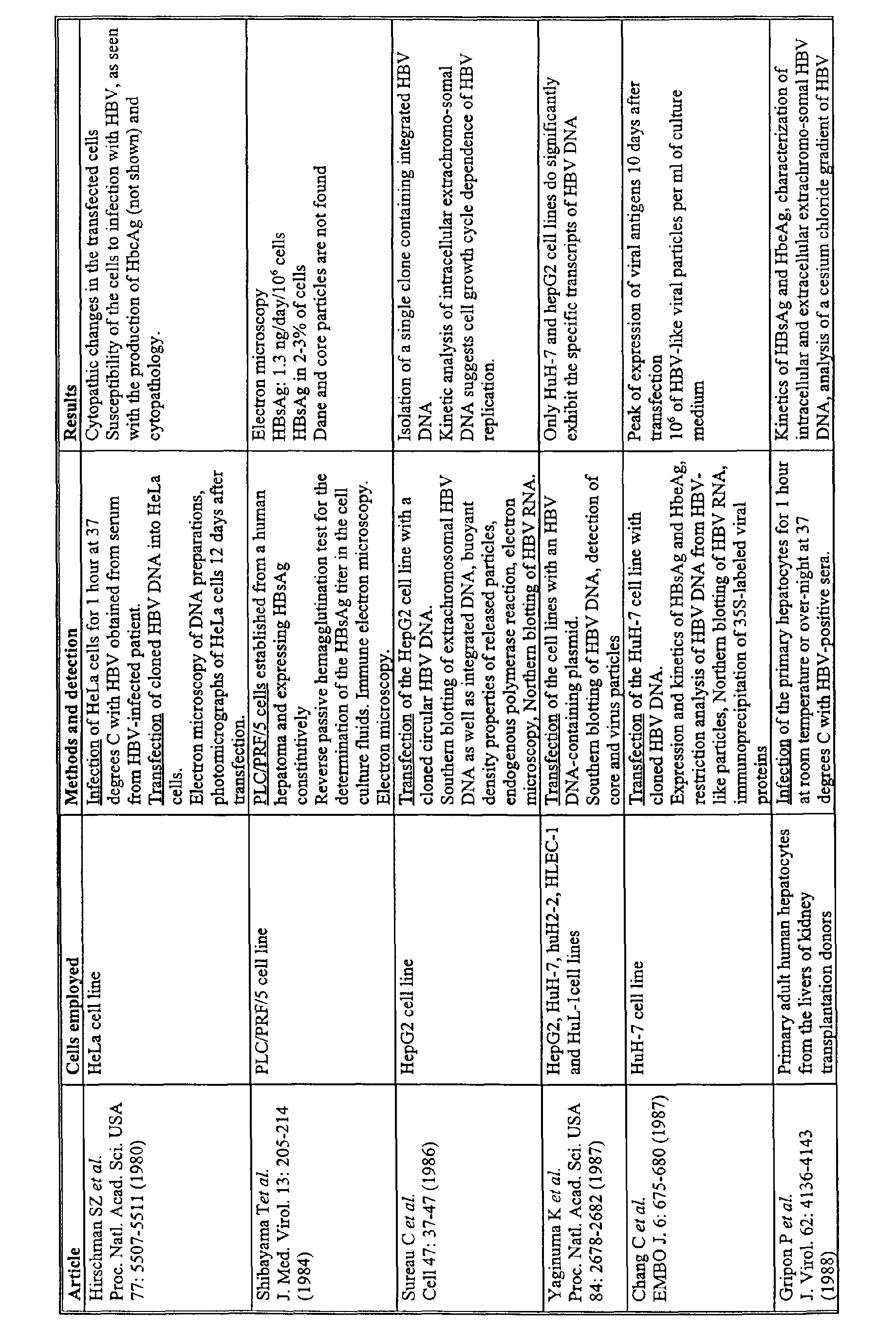

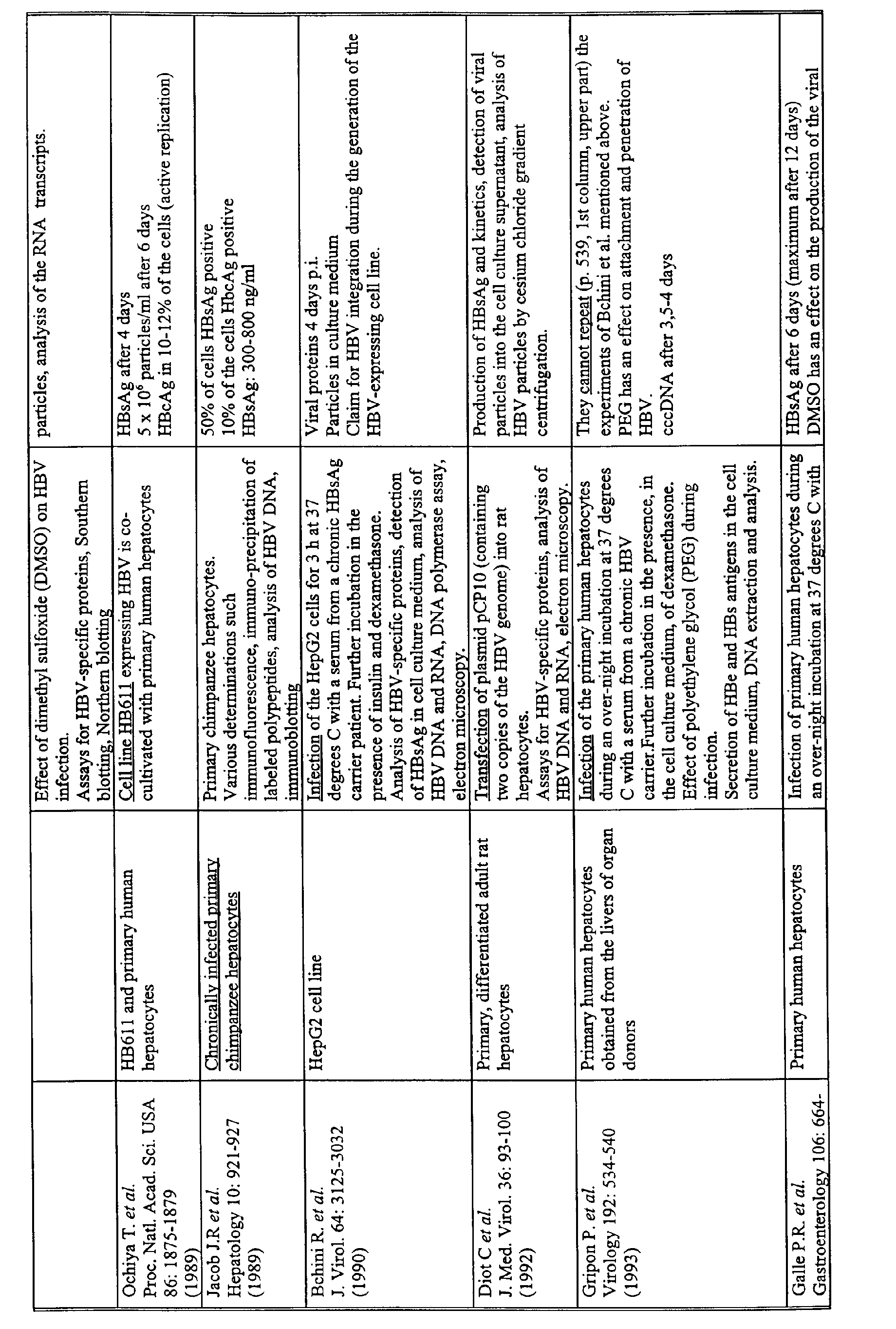

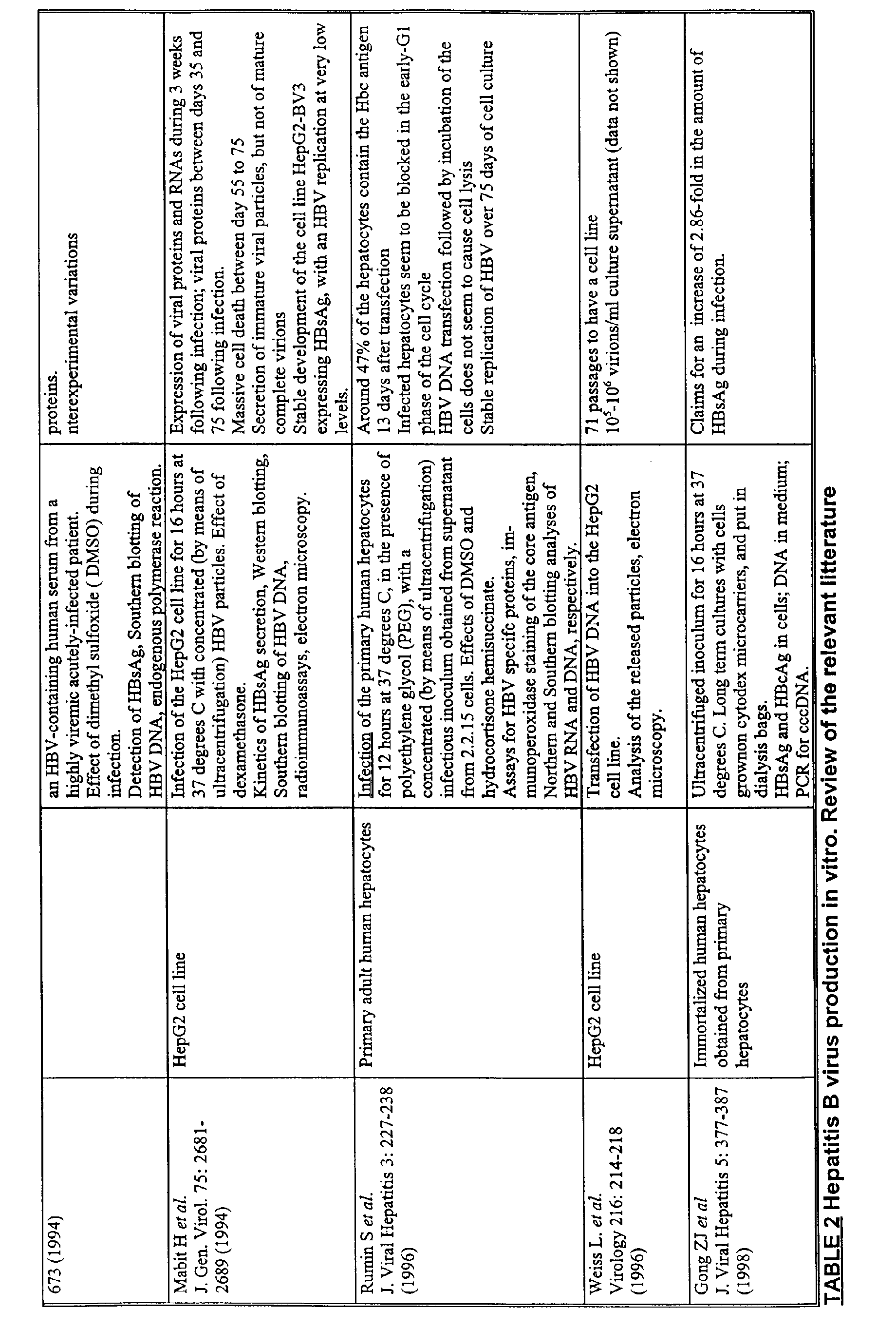

## Claims

1. Process for infecting eukaryotic cells with one or more virus species, characterized by the step of freeing the receptors for lipoproteins and/or lipids at the cell surface from essentially all lipoproteins and/or lipids prior to infection.

2. Process of claim 1, in which said virus species is/are one or several hepatitis-causative agents.

3. Process of claim 1, in which said virus species belong to the hepadnaviridae family.

4. Process of claim 3, in which said virus species is hepatitis B virus (HBV).

5. Process of claim 1, in which said virus species belong to the flaviviridae family.

6. Process of claim 5, in which said virus species is hepatitis C virus (HCV).

7. Process of claim 5, in which said virus species is the dengue virus.

8. Process of claim 5, in which said virus species is the yellow fever virus.

9. Process of claim 1 or 2, in which said virus species are hepatitis delta virus and hepatitis B virus.

10. The process of anyone of claims 1 to 9, characterized in that said eukaryotic cells are established hepatocyte cell lines.

11. The process of anyone of claims 1 to 9, characterized in that said eukaryotic cells are primary hepatocytes.

12. The process of anyone of claims 1 to 11, characterized in that said eukaryotic cells are of primate origin.

13. The process of anyone of claims 1 to 10, characterized in that said eukaryotic cells are the human HepG2 cell line.

14. The process of anyone of claims 1 to 10, characterized in that said eukaryotic cells are the human Huh-7 cell line.

15. The process of anyone of claims 1 to 9, characterized in that said eukaryotic cells naturally expresses a lipoprotein receptor.

16. The process of anyone of claims 1 to 9, characterized in that said eukaryotic cells expresses a lipoprotein receptor due to prior cloning of the gene encoding for a lipoprotein receptor.

17. The process of anyone of claims 1 to 16, characterized in that the freeing of the receptors for lipoproteins and/or lipids at the cell surface from essentially all lipoproteins and/or lipids is performed by adding to the culture medium, alone or in combination, one or more members of a group comprising dextran sulfate, heparin, ethylene glycol-bis (beta-aminoethyl ether) N,N, N', N'-tetraacetic acid (EGTA; ethylene-bis(oxyethylenenitrilo) tetraacetic acid; egtazic acid), ethylenediaminetetraacetic acid (EDTA; [ethylenedinitrilo] tetraacetic acid), or any other agent having similar properties.

18. The infected cell culture obtainable from the process of anyone of claims 1 to 17.

19. Use of the infected cell culture of claim 18 for producing antigens of said virus species.

20. Use of the infected cell culture of claim 18 for producing a vaccine against said virus species.

21. Use of the infected cell culture of claim 18 for producing a transformed cell line.

22. Use of the infected cell culture of claim 18 for producing a cell-based diagnostic assay.

23. Use of the infected cell culture of claim 18 for the screening of antiviral agents against said virus species.
